# EUROPEAN PATENT SPECIFICATION

(11) **EP 3 194 738 B1**
(45) Date of publication and mention of the grant of the patent: **16.10.2019**
(21) Application number: 15738923.0
(22) Date of filing: 17.07.2015
(51) Int. Cl.: H01M 8/0606, B01D 53/94, C01B 3/06, C01C 1/08, C05C 9/00, C12M 1/40, C12M 1/00, F01N 3/20, C01B 3/04, H01M 8/06, C05G 3/00

(54) **MODULE FOR DECOMPOSING AN AMMONIA PRECURSOR USING A CATALYST FOR USE ON-BOARD A VEHICLE**
MODULE ZUR ZERSETZUNG EINES AMMONIAKVORLÄUFERS MITTELS EINES KATALYSATORS ZUR VERWENDUNG IN EINEM FAHRZEUG
MODUEL DE DÉCOMPOSITION DE PRÉCURSEUR D'AMMONIAC À L'AIDE D'UN CATALYSATEUR DESTINÉ À ÊTRE UTILISÉ DANS UN VÉHICULE

(30) Priority: 18.07.2014 EP 14177713
(43) Date of publication of application: 26.07.2017
(73) Proprietor: Plastic Omnium Advanced Innovation and Research, 1130 Bruxelles (BE)
(72) Inventor: DOUGNIER, François, B-3191 Hever (BE); DE MAN, Pierre, B-1090 Bruxelles (BE); MONGE-BONINI, Beatriz, B-1040 Bruxelles (BE); VAN SCHAFTINGEN, Jules-Joseph, B-1300 Wavre (BE)
(74) Representative: Remy, Vincent Noel Paul
(86) International application number: PCT/EP2015/066496
(87) International publication number: WO 2016/009082

(56) References cited:
- WO-A1-2006/087541
- WO-A1-2007/099372
- WO-A1-2009/014516
- US-A1- 2009 120 078
- US-A1- 2012 183 281

## Description

### Field of Invention

The invention relates to a module for use on-board a vehicle, for decomposing an ammonia precursor solution using a catalyst, to a tank comprising such a module, to an SCR system comprising such a module and to a fuel cell system comprising such a module.

### Background

There exist prior art systems for supplying ammonia to an exhaust line of a vehicle in order to reduce the NOx emissions. A SCR (Selective Catalytic Reduction) process is used for converting nitrogen oxides of an exhaust gas coming from a vehicle engine into diatomic nitrogen and water. The SCR process enables the reduction of nitrogen oxides by injection of a reducing agent, generally ammonia, into the exhaust line. This ammonia may be obtained by using different techniques.

One known technique is based on the use of an ammonia precursor, for example an aqueous urea solution. Generally, such urea solution is stored in a tank mounted on the vehicle. A catalyst is used to generate ammonia from an ammonia precursor solution. Such systems are disclosed in patent applications WO 2015032811 and WO 2014095894, in the name of the Applicant, the content of which is included herein by reference.

WO 2006087541 discloses a device for generating gaseous ammonia formed by the hydrolysis of an aqueous solution of urea at elevated temperature and pressure and for feeding the gaseous ammonia into the exhaust gas on an internal combustion engine.

### Summary

The object of embodiments of the invention is to provide a multi-functional module which reduces the heat consumption needed for the decomposition from the ammonia precursor solution using the catalyst.

According to a first aspect of the invention there is provided a module for use on-board a vehicle according to appended claim 1.

The module comprises a heater and at least a first and a second storage compartment. The first compartment at least partially surrounds the heater, and the second compartment at least partially surrounds the first compartment. The first compartment is configured to perform a first function in a first temperature range, and the second compartment is configured to perform a second function in a second temperature range, said second temperature range being lower than said first temperature range. The first compartment is in fluid communication with the second compartment. One function of said first and second function is receiving an ammonia precursor, and decomposing the ammonia precursor using a catalyst to generate an ammonia solution.

By having at least two compartments arranged around a heater, the design and volume of those compartments can be optimized to obtain the appropriate temperatures in the compartments for decomposing the ammonia precursor and for input and/or output buffering or for pre- or post treatment, whilst reducing the required heat compared to a prior art embodiment where adjacent compartments are used. If the other compartment is merely a buffer compartment it is preferably the second compartment. If the other compartment is a separator compartment, the other compartment is preferably the first compartment as separating typically requires a higher temperature than decomposing.

Preferred embodiments are disclosed in the dependent claims.

In a preferred embodiment the other function of said first and second function is holding the ammonia precursor solution before it enters the compartment performing said one function and/or holding the generated ammonia solution leaving said compartment performing said one function. In other words the other function may be a pre- or post-buffer function of a pre- or post treatment function, such as a separation function.

In a preferred embodiment the heater extends in the first compartment, preferably centrally in the first compartment. In that way a good heat distribution can be obtained in the first compartment, wherein the heat is further spread from the first compartment to the second compartment

In a preferred embodiment the first compartment has an outer circumferential wall, e.g. a cylindrical outer wall, and the second compartment surrounds the outer circumferential wall of the first compartment. The outer circumferential wall of the first compartment may correspond with an inner circumferential wall of the second compartment.

According to an exemplary embodiment the generated ammonia solution comprises ammonia, carbon dioxide and water, said one decomposing function is the second function, and the first function is separating the generated ammonia solution. Preferably the generated ammonia solution is separated into a first ammonia rich fraction and a carbon dioxide rich fraction; said first ammonia rich fraction containing a smaller weight percentage of carbon dioxide than said solution and said carbon dioxide rich fraction containing a smaller weight percentage of ammonia than said solution. Such a separation process typically requires higher temperatures than the decomposing process. Therefor it is advantageous to perform the separation in the first compartment and the decomposing in the second compartment. A possible separation process is disclosed in European patent application EP 14163101.0 in the name of the Applicant.

In a further developed embodiment thereof the module further comprises an output buffer compartment surrounding at least partially the second compartment, said output buffer compartment being in fluid communication with the first compartment and being configured for holding the ammonia rich fraction leaving said first compartment. Alternatively or in addition the module may further comprise an input buffer compartment surrounding at least partially the second compartment, said input buffer compartment being in fluid communication with the second compartment and being configured for holding the ammonia precursor solution before entering the second compartment. The input buffer may be surrounding the output buffer or vice versa. Alternatively one surrounding compartment may comprise an input buffer chamber and an output buffer chamber, see e.g. figure ID.

In an alternative embodiment said one decomposing function is the first function performed in the first compartment, and said second function performed in the second compartment is a buffer function. This may be an input buffer function for ammonia precursor solution, or an output buffer function of ammonia solution.

According to the invention the module further comprises a valve block configured for connecting at least said first and second compartment. Also the module comprises a control module configured for controlling the valve block and the heater such that said one function comprises receiving a fresh ammonia precursor solution whilst outputting a generated ammonia solution, at repetitive moments in time; and such that said other function comprises receiving the ammonia precursor solution before it enters said one compartment and/or receiving the generated ammonia solution leaving said one compartment.

In a preferred embodiment with separating function, the control module is configured for controlling the valve block and the heater such that the ammonia solution in said first compartment is heated at a first temperature (T3); and the ammonia precursor solution in said second compartment is heated at a second temperature (T1) lower than said first temperature. Said second temperature may be in a range between 40 and 60 degrees Celsius and said first temperature may be in a range between 70 and 95 degrees Celsius, preferably between 80 and 90 degrees Celsius.

In a preferred embodiment with output buffer compartment, the valve block is configured for connecting the output buffer compartment with the first compartment; and /or for connecting the input buffer compartment with the second compartment. More generally the valve block may be configured for connecting the various compartments as needed, wherein one or more pumps or other transfer means may be added for transferring the fluids from one compartment to another or to or from an ammonia precursor tank.

The control module may be configured for controlling said valve block and said one or more pumps or other transfer means. In a particular embodiment the control module is configured to control the valve block such that after a separating and decomposing step, an ammonia rich fraction is transferred from the first compartment to the output buffer compartment, an ammonia solution is transferred from the second compartment to the first compartment, and an ammonia precursor solution is transferred to the second compartment. The control module may be further configured for controlling said valve block such that after a separating and decomposing step, an ammonia precursor solution is transferred to the input buffer compartment, and an ammonia precursor solution is transferred from said input buffer compartment to said second compartment. In a preferred embodiment each compartment has a cylindrical inner and outer wall. Preferably the first and second compartments are concentric around the heater.

A typical application of embodiments of the invention is for the conversion of ammonia precursor into ammonia using enzymes as a catalyst. The generated ammonia is used for Selective Catalytic Reduction (SCR) of the exhaust gases (NOx reduction), as fuel for fuel cells, or for other applications on-board of the vehicle.

In a preferred embodiment the ammonia precursor solution is AdBlue®, and the catalyst is adapted for decomposing the ammonia precursor solution to generate ammonia. In a preferred embodiment the catalyst is a biological catalyst. The biological catalyst is preferably a catalyst composition in a solid form, e.g. enzymes in solid form. Preferably the enzymes are immobilized on a carrier. Enzymes may be immobilized on a variety of carriers including synthetic polymers, biopolymers, hydrogels and inorganic supports. Examples of biopolymers are for instance gelatin, polysaccharides such as cellulose, starch, agarose, chitosan and alginates. Examples of inorganic supports are alumina, silica, zeolites and mesoporous silica. One suitable method is the immobilization by silica granulation. This may result in gradual removal of the enzyme from the carrier. Alternatively, immobilization is achieved by means of entrapment in sol-gel matrices, such as silica sol-gels prepared by hydrolytic polymerisation of tetraethoxysilane and other silanes. Modified sol-gels are also known as xerogels, ambigels and aerogels. The sol-gels may be designed to exhibit large surface areas and pore volumes, with hollow spherical morphology, as well as hydrophilic surfaces. The capsules and/or other solid forms are preferably provided with a diameter in the range of 10 microns to 10 cm, preferably 1 mm to 1 cm. The capsule may be based on microencapsulation, comprising a plurality of beads with enzyme immobilized on carrier in a matrix. The matrix may be herein optimized to be slowly dissolvable, in dependence of the pH of the reaction medium, so as to obtain modified-release capsules. In a preferred embodiment, use is made of a capsule that does not - or at least not substantially - disintegrate. The carrier is preferably a porous body, and the enzyme is suitably located at a surface inside said porous body. In an exemplary embodiment, the catalyst is an urease enzyme. This catalyst is suitable for the conversion of an ammonia precursor such as urea. Suitably, the urease enzyme is immobilized on a biopolymer carrier. The use of such a carrier results in improved stability. One example of a suitable solid carrier is for instance a synthetic polymer such as polystyrene, EVOH, nylon-6 and the like. Other examples of carriers are carbohydrates, such as chitosan, dextran and agarose; porous nanoparticles, for instance of silica; gelatine; and alginates. The use of chitosan is deemed highly suitable, since it tends to increase optimum pH of the urease to a higher pH, resulting in optimum activity in the ammonia precursor composition e.g. solution. In an exemplary embodiment the catalyst is provided in solid form. The solid form may be a powder or a powder compressed into one or more pellets, granules or beads. Alternatively, the catalyst may be provided in the form of capsules, pills or cartridges. In other exemplary embodiments the catalyst may be provided in liquid form. In a particular embodiment, the catalyst may be provided as a liquid composition inside a solid jacket such as a cartridge, a capsule, etc. In an exemplary embodiment the catalyst (solid or liquid) may be encapsulated. In a first exemplary embodiment, the catalyst may be provided with a coating to maintain integrity and to avoid contamination of the catalyst and/or of its composition. In another exemplary embodiment the catalyst may be provided with a hydrogel encapsulating the catalyst. In an exemplary embodiment, use is made of a solid substrate onto which the catalyst is immobilized. Examples of suitable solid substrates are: synthetic polymers, such as polystyrene, EVOH, nylon-6 and the like; carbohydrates, such as chitosan, dextran and agarose; porous nanoparticles, for instance of silica.

According to another aspect of the invention according to appended claim 16 there is provided a tank comprising a module of any one of the above disclosed embodiments, said module being integrated in the tank or in or on a wall of the tank. This may be e.g. a bottom wall or a top wall. The module may be configured for allowing ammonia precursor solution stored in the tank to be transferred to one compartment of said first and second compartment, optionally via the other compartment of said first and second compartment. In a preferred embodiment the module comprises an inlet opening near the bottom of the module and the module is arranged in the tank.

According to another aspect of the invention according to claim 17 there is provided an SCR system for a vehicle comprising a module according to any one of the embodiments disclosed above.

According to another aspect of the invention according to appended claim 18 there is provided a fuel cell system comprising a module according to any one of the embodiments disclosed above.

### Brief description of the figures

The accompanying drawings are used to illustrate presently preferred non-limiting exemplary embodiments of devices of the present invention. The above and other advantages of the features and objects of the invention will become more apparent and the invention will be better understood from the following detailed description when read in conjunction with the accompanying drawings, in which:
Figures 1A, 1B, 1C and 1D illustrate schematic perspective views of four exemplary embodiments of a module of the invention;
Figures 2A, 2B, and 2C illustrate schematic cross sections of three exemplary embodiments of a tank with integrated module according to the invention;
Figure 3 is a schematic cross section of an exemplary embodiment of a module of the invention with four compartments;
Figure 4 is a schematic cross section of an exemplary embodiment of a module of the invention with three compartments;
Figure 5 is a schematic cross section of an exemplary embodiment of a module of the invention with an enlarged output buffer;
Figure 6 is a schematic cross section of an exemplary embodiment of a tank with a module of the invention and an additional storage capacity;
Figure 7 is a schematic cross section of an exemplary embodiment of a module of the invention with additional enzyme storage.

### Description of embodiments

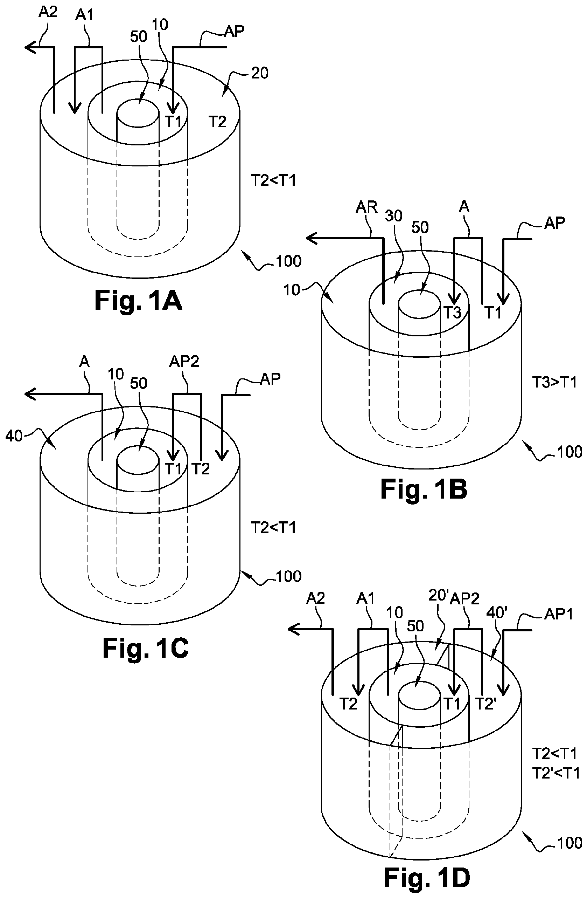

Figure 1A illustrates an exemplary embodiment of a module 100 for use on-board a vehicle, for decomposing an ammonia precursor AP using a catalyst. The module 100 comprises a heater 50, a first storage compartment 10 and a second storage compartment 20. The first compartment 10 is arranged around the heater 50, and the second compartment 20 is arranged around the first compartment 10, such that, when said heater 50 is operated the first and second compartments are at decreasing temperatures T1, T2 with T2<T1. The first compartment 10 is configured for receiving an ammonia precursor solution AP, and for decomposing the ammonia precursor solution AP using the catalyst and heat from heater 50 to generate an ammonia solution A1. The second compartment 20 is in fluid communication with the first compartment 10 and is configured for holding the generated ammonia solution A1 leaving the first compartment 10. The second compartment 20 functions as an output buffer compartment. When ammonia solution is needed, e.g. for feeding a fuel cell or an SCR system, it can be transferred from the output buffer compartment 20 to the fuel cell or SCR system, see arrow A2.

Figure 1B illustrates another exemplary embodiment of a module 100 for use on-board a vehicle, for decomposing an ammonia precursor AP using a catalyst. The module 100 comprises a heater 50, a first storage compartment 30 and a second storage compartment 10. The first compartment 30 is arranged around the heater 50, and the second compartment 10 is arranged around the first compartment 30, such that, when said heater 50 is operated the first and second compartments are at decreasing temperatures T3, T1 with T1 < T3. The second compartment 10 is configured for receiving an ammonia precursor solution AP, and for decomposing the ammonia precursor solution AP using the catalyst and heat from heater 50 to generate an ammonia solution A. The generated ammonia solution comprises ammonia, carbon dioxide and water. The first compartment 30 is configured for separating the generated ammonia solution A into a first ammonia rich fraction and a carbon dioxide rich fraction; said first ammonia rich fraction AR containing a smaller weight percentage of carbon dioxide than said solution and said carbon dioxide rich fraction containing a smaller weight percentage of ammonia than said solution. When said fraction AR is needed, e.g. for feeding a fuel cell or an SCR system, it can be transferred from the first compartment 30 to the fuel cell or SCR system. In the illustrated embodiment the first compartment has a single outlet. However, a skilled person understands that two outlets are also an option. The number of outlets typically depends on the separation process which is implemented. If one single outlet is used, e.g. CO2 may be first eliminated (as gas) and next the remaining NH3 solution may be transferred through the same outlet.

Figure 1C illustrates an exemplary embodiment of a module 100 for use on-board a vehicle, for decomposing an ammonia precursor AP using a catalyst. The module 100 comprises a heater 50, a first storage compartment 10 and a second storage compartment 40. The first compartment 10 is arranged around the heater 50, and the second compartment 40 is arranged around the first compartment 10, such that, when said heater 50 is operated the first and second compartments are at decreasing temperatures T1, T2 with T2<T1. The second compartment 40 is configured for holding ammonia precursor solution AP1 and is in fluid communication with the first compartment 10, see arrow AP2. The second compartment 40 functions as an input buffer compartment. The first compartment 10 is configured for receiving an ammonia precursor solution AP2, and for decomposing the ammonia precursor solution AP2 using the catalyst and heat from heater 50 to generate an ammonia solution A. When ammonia solution A is needed, e.g. for feeding a fuel cell or an SCR system, it can be transferred from the first compartment 10 to the fuel cell or SCR system.

Figure ID illustrates an exemplary embodiment of a module 100 for use on-board a vehicle, for decomposing an ammonia precursor AP using a catalyst. The module 100 comprises a heater 50, a first storage compartment 10 and a second storage compartment with two sub-compartments 20', 40'. The first compartment 10 is arranged around the heater 50, and the second sub-compartments 20', 40' are arranged around the first compartment 10, such that, when said heater 50 is operated the first and second compartments are at decreasing temperatures T1, T2/T2' with T2,T2'<T1. The second sub-compartment 40' is configured for holding ammonia precursor solution AP1 and is in fluid communication with the first compartment 10, see arrow AP2. The second sub-compartment 40' functions as an input buffer compartment. The first compartment 10 is configured for receiving the ammonia precursor solution AP2 from the input buffer compartment 40', and for decomposing the ammonia precursor solution AP2 using the catalyst and heat from heater 50 to generate an ammonia solution A1. The second sub-compartment 20' is in fluid communication with the first compartment 10 and is configured for holding the generated ammonia solution A1 leaving the first compartment 10. The second sub-compartment 20' functions as an output buffer compartment. When ammonia solution is needed, e.g. for feeding a fuel cell or an SCR system, it can be transferred from the output buffer compartment 20' to the fuel cell or SCR system, see arrow A2.

Although not shown in figures 1A-1D, the skilled person understands that the module 100 further comprises a valve block configured for connecting the first and second compartment, and a control module configured for controlling the valve block and the heater. Preferably the controlling is such that compartment 10 receives a fresh ammonia precursor solution whilst outputting a generated ammonia solution, at repetitive moments in time, and such that compartment 20, 20', 30 receives the ammonia precursor solution before it enters compartment 10 or that compartment 40, 40' receives the generated ammonia solution leaving compartment 10.

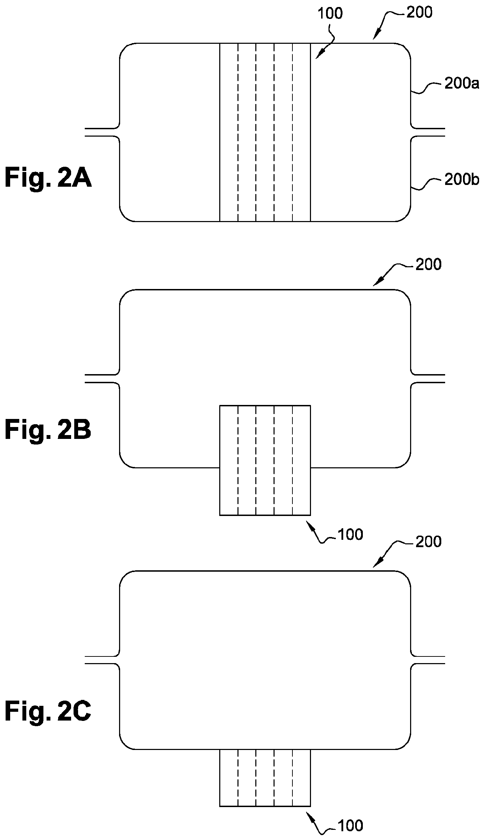

Figures 2A, 2B and 2C illustrate that the module 100 may be integrated in a tank 200 (figure 2A) or in or on a wall of the tank 200. This may be a bottom wall, a top wall or a side wall of the tank 200. The module 100 is configured for allowing ammonia precursor solution stored in tank 200 to be transferred either directly to compartment 10 (embodiment of figures 1A and 1B) or to an input buffer compartment 40, 40' (embodiment of figures 1C and ID).

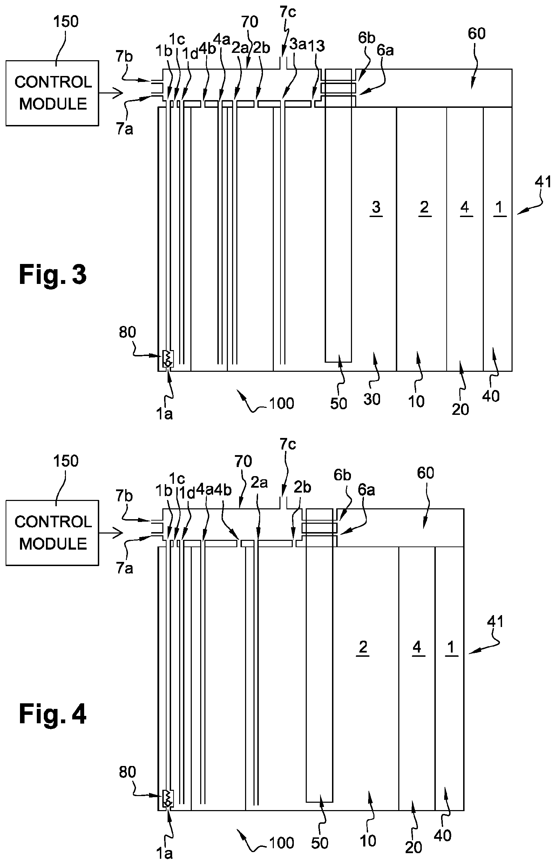

Figure 3 illustrates an exemplary embodiment of a module 100 of the invention with four compartments 30, 10, 20, 40 surrounding the heater 50. The compartments 30, 10, 20, 40 correspond with four circular, concentric chambers 3, 2, 4, and 1. The module 100 is intended for being mounted in or adjacent a tank (not shown in figure 3) filled with an ammonia precursor solution, typically an Adblue® fluid matching the ISO 22241 standard specification and containing 32.5 ± 0.7 weight % urea.

The outer compartment 40 is delimited by a cylindrical outer wall 41 and a cylindrical inner wall 42. The cylindrical outer wall 41 has a diameter which is larger than the diameter of the cylindrical inner wall 42. The outer compartment 40 corresponds with chamber 1 which consists in an input buffer receiving ammonia precursor solution from the tank.

The ammonia precursor solution in chamber 1 is carried to chamber 2 which corresponds with the second compartment 10. The second compartment 10 has a cylindrical outer wall 11 and a cylindrical inner wall 12. The cylindrical outer wall 11 has a diameter which is larger than the diameter of the cylindrical inner wall 12. Chamber 2 is used as a conversion unit for the ammonia precursor solution, using a catalyst, e.g. a biological catalyst such as an enzyme, to produce effluents comprising ammonia. In the example of an enzyme, the enzyme may be immobilized in granular form and may be introduced in chamber 2 through a cap (not illustrated), e.g. on the top or the bottom of the module.

The effluents from chamber 2 are carried into chamber 3 which corresponds with the first compartment 30. The first compartment has a cylindrical outer wall 31. The heater 50 is arranged centrally in the first compartment 30. Chamber 3 works as a separation unit. Separation may comprise transforming the effluents (produced in chamber 2) in an aqua ammonia solution with a low concentration of carbonates.

The aqua ammonia solution with low concentration of carbonates is carried in chamber 4 which corresponds with a third compartment 20. The third compartment 20 has a cylindrical outer wall 21 and a cylindrical inner wall 22. The cylindrical outer wall 21 corresponds with the inner wall 42 of the outer compartment 40. The cylindrical inner wall 22 corresponds with the outer wall 11 of the second compartment 10. Chamber 4 is a buffer to store the effluents after the separation step.

The heater 50 may be a tubular heater inserted along the axis of the chambers.

In the illustrated embodiment a pump 60 and a flow distribution multivalve 70 are located on the top of the module. The pump 60, heater 50 and flow distribution multivalve 70 are connected to an engine control unit (ECU, not indicated on the drawing).

As an advantage, the module of figure 3 presents an optimized heat transfer, with minimal heat losses. The chambers 1, 2, 3, and 4 (i.e. the compartments 10, 20, 30, 40) and the heater 50 are designed so that the fluid temperature T1 in chamber 2 corresponds to the required temperature for catalyst activity and the fluid temperature in chamber 3 corresponds with a suitable temperature T3 (T3 > T1) for the separation process. In a preferred embodiment where enzymes are used as the catalyst, heater 50 and compartments 10, 20, 30, 40 are designed for causing a temperature T1 suitable for enzymatic activity, e.g. within a 40°C - 60°C temperature range. Temperature T3 in chamber 3 may be e.g. between 70°C and 95°C.

Chambers 1 and 4 at the periphery of the module 100 act as a thermal buffer, when the device is activated, thus limiting the power needed for heating chambers 3 and 2. For a supplemental thermal insulation, the outer periphery 41 of the module 100 may be moulded as a double wall, such that an air gap is formed creating an additional insulation layer. Also, PCM materials may be used to further maintain the temperature at the operating conditions, at the end of a heating phase.

The module 100 further comprises a check valve 80 which prevents chamber 1 from emptying when the liquid level in the tank is lower than the liquid level in chamber 1.

The design of figure 3, when the module is placed in the tank, also provides intrinsic safety as regards the risks associated with ammonia solutions. If a leak occurs in chamber 2, 3 or 4, the content of these chambers 2, 3 or 4 is to be diluted with the ammonia precursor solution inside the tank, thus avoiding any waste outside the vehicle. The outlet of the venting pathways of the module 100 is located also inside the tank, meaning that the ammonia gas atmosphere which is potentially generated in chamber 3 during the separation process is trapped in the ammonia precursor solution, and not rejected in the environment of the vehicle.

The compartments 10, 20, 30, 40 of module 100 may be moulded in plastic material, e.g. PA, PA66, PPA, POM. The connection between the module 100 and the tank may be a mason jar-type or a cam-lock type connection. As explained with reference to figures 2A-2C, the module 100 may be suitably mounted on or in the top of the tank, entirely inside the tank, on or in the bottom of the tank.

### Module Operation

The initiating phase, i.e. the first use of the module 100, goes through the following steps:
A - The ammonia precursor solution is pumped from the tank through inlet 1a in chamber 1. Port 1b of the distribution multivalve 70 is connected to port 2a, port 2b is connected to port 3a, port 3b is connected to pump inlet 6a, and pump outlet 6b is connected to valve outlet 7a. Pump 60 is operated in order to fill subsequently chambers 2 and 3. Venting is provided through outlet 7a. The heater 50 is powered in order to get the required temperatures in chambers 3 and 2. Filling chamber 3 with ammonia precursor solution enhances the thermal transfer from heater 50 to chamber 2.
B - When the enzymatic conversion is completed in chamber 2, the ammonia precursor solution in chamber 3 is evacuated. The multivalve 70 provides fluid connection between port 3a and pump inlet 6a, between pump outlet 6b and valve outlet 7a. Port 7b is connected to port 3b. Thus, the ammonia precursor solution in chamber 3 is sent back to the tank, and venting is provided via a pathway between ports 7a and 3b.
C - The generated ammonia solution in chamber 2 is transferred to chamber 3. To achieve that, port 2a is in fluid communication with the pump inlet 6a, and pump outlet 6b is connected to port 3a. Chamber 2 is vented through the connection of inlet 7a to port 2b. Venting of chamber 3 occurs by connecting port 3b to valve outlet 7b.
D - Chamber 2 is filled again with ammonia precursor solution. For that, port 1a is connected to pump inlet 6a, pump outlet 6b to port 2a, and chamber 2 is vented through port 2b connected to valve inlet 7a. When powering the heater 50, the separation process takes place in chamber 3, whilst ammonia precursor conversion occurs in the chamber 2.
E - When separation is achieved in chamber 3 and enzymatic conversion is completed in chamber 2, the effluents in chamber 3 are transferred to chamber 4: port 3a is connected to pump inlet 6a, pump outlet 6b is connected to port 4a; chamber 3 is vented by connecting port 3b to port 7a and chamber 4 is vented through the connection between port 4b and valve outlet 7b.

When required by the De-NOx function of the selective catalytic reduction (SCR) system, the reductant solution in chamber 4 is transported to the exhaust line, by connecting port 4a to pump inlet 6a and pump outlet 6b to valve outlet 7c. Chamber 4 is vented by connecting valve inlet 7a to port 4b.

After the initiating phase, the module works as follows: converted ammonia precursor solution in chamber 2 is transferred to chamber 3 according to step C. Chamber 2 is re-filled with ammonia precursor solution according to step D. Chamber 1 is also filled with ammonia precursor solution by connecting port 1b to pump inlet 6a and pump outlet 6b to port Id. Ammonia precursor solution is sucked through inlet 1a and chamber 1 is vented by connecting port 1c to valve outlet 7b. This provides a pre-heating of the ammonia precursor solution in chamber 1 during the conversion step occurring in chamber 2 and the separation step occurring in chamber 3, when heater 50 is operated. When the decomposition reaction and the separation process are completed, the content of chamber 3 is transferred to chamber 4, the content of chamber 2 is transferred to chamber 3, and chamber 2 is filled with the content of chamber 1. Chamber 1 is re-filled with ammonia precursor solution from the tank.

The module 100 further comprises a control module 150 configured for controlling the valve block 70, the pump 60, and the heater 50 such that the above described mode of operation is performed. More in particular the control module 150 is configured for controlling the valve block 70, the pump 60 and the heater 50 such that the ammonia solution in said first compartment 30 is heated at a first temperature, and the ammonia precursor solution in the second compartment 10 is heated at a second temperature lower than said first temperature. The control module 150 is configured for controlling said valve block 70 such that after a separating and decomposing step, an ammonia rich fraction is transferred from the first compartment 30 to the output buffer compartment 20, an ammonia solution is transferred from the second compartment 10 to the first compartment 30, an ammonia precursor solution is transferred to the input buffer compartment 40 (from a tank), and an ammonia precursor solution is transferred from said input buffer compartment 40 to said second compartment 10.

In the mode of operation explained above, the module 100 works in a batch mode. In another embodiment the module may work with a continuous flow. The module 100 can be part of an SCR system. Alternatively or in addition, the module 100 can also be used to feed a fuel cell.

Figure 4 illustrates another exemplary embodiment of a module 100 designed for a system with no need of further effluents separation after the catalytic conversion of the ammonia precursor solution, e.g. Adblue®. The module 100 is similar with this difference that compartment 30 has been omitted. The same reference numerals have been used to indicate the same or similar components. The module 100 has three chambers 1, 2 and 4: chamber 1 as an input buffer, chamber 2 for ammonia precursor conversion, and chamber 4 for storage of the generated ammonia solution coming from chamber 2. A catalyst, e.g. an enzyme, is inserted in chamber 2, partially filling its volume. The ammonia precursor solution is sucked by pump 60, through inlet 1a and multivalve 70, and the enzymatic reaction is thermally activated with heater 50. After full conversion, the effluents are transferred into chamber 4. Chamber 2 is now ready for refilling with urea solution and further conversion. The effluents in chamber 4 may be sent to an exhaust line through the outlet 7c. The functioning of the module is basically the same as the one which is described in figure 3, except for the transfers from and to the separation chamber 3. Chamber 1 is used for pre-heating the ammonia precursor solution at a suitable temperature for the catalytic conversion.

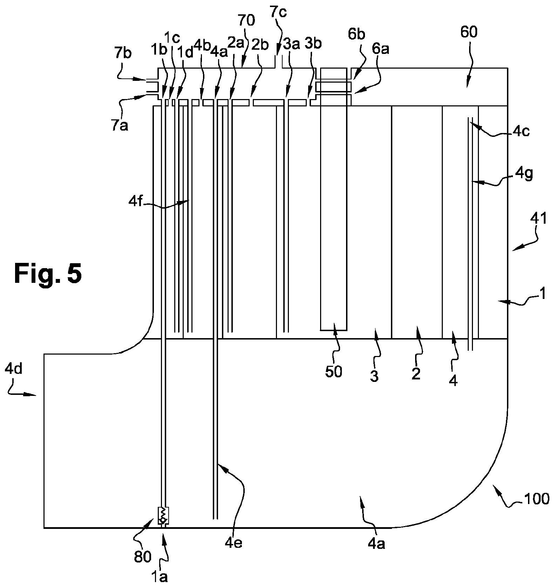

Figure 5 illustrates an embodiment which is similar to the module 100 of figure 3, except that a larger volume is provided for the storage of the separated effluents. The design of the output buffer consists of two chambers 4 and 4' in fluidic communication through the tube 4g. After separation, the effluents are transferred from chamber 3 to chamber 4, by connecting the port 3a to the port 4b via pump 60, and venting chamber 4 through the tube 4g, chamber 4' and tube 4e, with a connection between the port 4a and the valve outlet 7b. When the liquid level in chamber 4 reaches orifice 4c of tube 4g, the solution in chamber 3 is transferred to the chamber 4' by connecting port 3a to port 4a. The venting path of the chamber 4' is achieved through the tube 4g, and the connection of the port 4b to the valve outlet 7b. Tube 4f is used to pump the separated effluents in the chamber 4.

The design of the module 100 of figure 5 allows an easy insertion of the module 100 inside a tank, especially in a tank with a limited height and a limited diameter of the opening in the tank wall for mounting the module; first side 4d is inserted through the opening in the tank wall, and then the rest of module 100 is inserted whilst operating a pivotal movement during assembly.

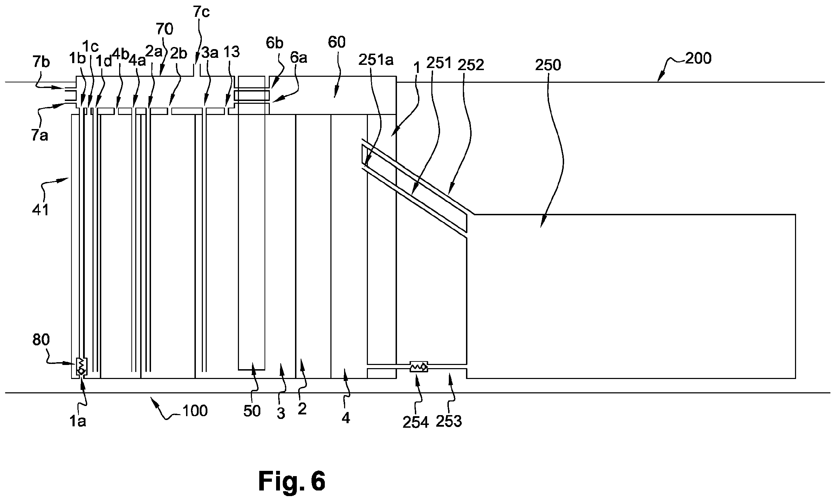

Figure 6 illustrates an exemplary embodiment of a tank 200 in which a module 100 similar to the module of figure 3, is arranged. In this embodiment, chamber 4 of the module 100 is in fluidic connection with a storage capacity 250, inserted in the ammonia precursor tank 200 and external to the module 100. The volume of the storage capacity 250 may be e.g. up to 20 to 40% of the volume of the tank 200. The storage capacity 250 may have a variable volume, working as a bladder tank. Depending on the conversion rate of the catalyst, and the efficiency of the separation process, this storage capacity 250 may be integrated in the module. Also, the size of the chambers 2 and 3 may be reduced to allow for such integration. The module 100 works in a similar way as the module 100 of figure 5. The separated effluents flow to storage capacity 250 when the liquid level reaches opening 251a of line 251 connecting chamber 4 with storage capacity 250. The storage capacity 250 is vented via line 252 with port 4b connected to valve outlet 7b. Check valve 254 on line 253 is implemented so that chamber 4 can be filled with the solution coming from chamber 3, which constitutes an advantage for heat transfer, as chamber 4 is then not anymore an air gap. When the injection of the ammonia solution is required in the SCR system or when feeding a fuel cell is required, the separated effluents in storage capacity may be sucked by pump 60 in chamber 4 through port 4a. The effluents in the capacity storage 250 are transferred into chamber 4 through the line 253.

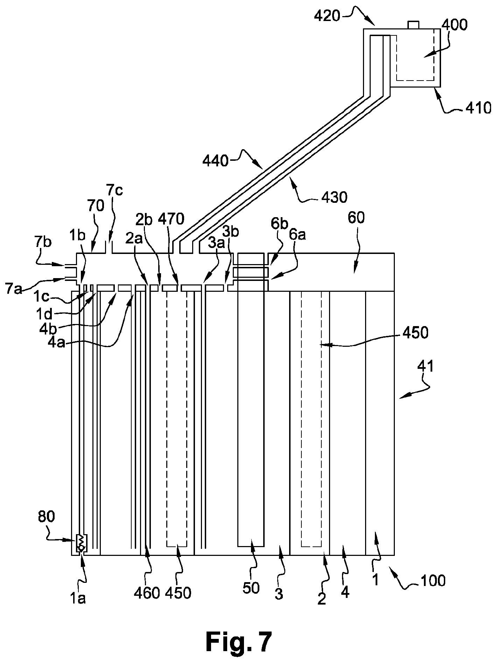

Figure 7 illustrates a further variant of a module 100 which is similar to the module of figure 3. The module 100 of figure 7 provides an improved way of adding an immobilized catalyst, in particular immobilized enzymes, in chamber 2, when the top of the module 100 is not easily accessible due to the integration of the SCR system in a vehicle. In this embodiment, the enzymes are enclosed in a strainer 400 which is introduced in a storage capacity 410. Storage capacity 410 may be conveniently located in the vehicle, so that the strainer 400 is easily inserted and removed. The strainer 400 is connected to flow distribution multivalve 70 through an outlet 420. Chamber 2 is also equipped with a strainer 450. The enzyme is transferred from strainer 400 to strainer 450 by flushing with ammonia precursor solution. The ammonia precursor solution is pumped in chamber 2 from channel 460 to line 430 and flushes the enzyme in line 440 through outlet 420. The enzyme is introduced in strainer 450 through inlet 470. The enzyme transfer from the storage capacity 410 to the strainer 450 can be progressive or by steps, in order to partially refresh the catalyst for urea decomposition in chamber 2, which is an advantage as regards enzyme durability. After the required amount of enzyme is added to the strainer 450, the storage capacity 410 can be drained in order to further limit the contact with ammonia precursor solution and to increase the enzyme storage limit. The storage capacity 410 may be thermally protected, by implementing a thermal conditioning means, such as a heater, a cooler, a device based on the Peltier effect, or PCM materials. The storage capacity 410 could also be located in a thermally protected area in the vehicle. When the immobilized enzyme has to be removed from the strainer 450, because it has lost most of its activity, the content of strainer 450 is flushed to strainer 410 through line 440. After trapping the immobilized enzyme, the remaining fluid returns to chamber 2 through line 430. Preferably, the pumping time is chosen to be long enough in order to empty the immobilized enzyme from the strainer 450. The strainer 400 may be a cartridge or retaining unit containing the enzymes, e.g. as disclosed in EP 15 162 678.5 filed on 7 April 2015 in the name of the Applicant, the entire of which is included herein by reference.

An example of a urea decomposition system is disclosed in patent applications WO 2015032811 and WO 2014095894 in the name of the Applicant, the contents of which are included herein by reference. In those applications the Applicant has proposed two new methods for generating ammonia on board a vehicle (passenger car, truck, etc.) based on a biological catalysis. Biological catalysis comprises all forms of catalysis in which the activating species (i.e. biological catalysts) is a biological entity or a combination of such. Included among these are enzymes, subcellular organelles, whole cells and multicellular organisms. More precisely, according to a first method, a protein component is used to catalyse the hydrolysis (i.e. decomposition) of an ammonia precursor solution (for example, urea) into a mixture comprising at least ammonia, carbon dioxide and water. Such first method is described in more detail in patent application WO 2015032811. According to a second method proposed by the Applicant, a protein component is used to catalyse the hydrolysis (i.e. decomposition) of an ammonia precursor solution (for example, urea) into ammonia gas. For example, the generated ammonia gas can be directed (i.e. transmitted) to a solid absorbing matrix where it is stored thereon by sorption. Such second method is described in more detail in patent application WO 2014095894.

In embodiments of the invention the heater 50 heats up the decomposition area at the appropriate temperature for the reaction, i.e. for the decomposing of the ammonia precursor into ammonia. In the event that the biological catalyst is urease, a suitable temperature T1 would be from around 40 to 60°C. The heater 50 can be of any type as known in the state of the art. Typically a resistive heater is well suited. However, it is also possible to provide, as a heater, a conduit through which the cooling liquid of the engine is circulated.

The catalyst can have several shapes. In the case of enzymes, they can be powder, pellets, granules or beads; the enzymes can be immobilized or not on a substrate, and the substrate can be part of the chamber 2.

Embodiments of the invention may also be used in an ammonia precursor booster system comprising a storage compartment for storing ammonia precursor granules, and a dissolving compartment for storing an ammonia precursor solution, and for dissolving ammonia precursor granules in the ammonia precursor solution, a decomposition unit, and optionally one or more buffers. The dissolving compartment, the decomposition unit and the one or more buffers could be implemented as a module with a plurality of concentric compartments. An example of such a booster system is disclosed in European patent application EP 14177713 filed on 18 July 2014 in the name of the Applicant, the content of which is included herein by reference.

Also further developed embodiments of the module of the invention may comprise a conversion unit for converting ammonia into hydrogen, and a hydrogen fuel cell.

Whilst the principles of the invention have been set out above in connection with specific embodiments, it is to be understood that this description is merely made by way of example and not as a limitation of the scope of protection which is determined by the appended claims.

## Claims

1. A module (100) for use on-board a vehicle, said module comprising a heater (50), and at least a first and a second storage compartment (10; 20; 30; 40; 20',40');
wherein the first compartment at least partially surrounds said heater (50), and the second compartment at least partially surrounds the first compartment;
the first compartment being for performing a first function in a first temperature range, and the second compartment being for performing a second function in a second temperature range, said second temperature range being lower than said first temperature range;
wherein the first compartment is in fluid communication with the second compartment;
wherein one function of said first or second function is decomposing an ammonia precursor using a catalyst to generate an ammonia solution;
wherein the module further comprises a valve block (70) configured for connecting at least said first and second compartment, and a control module (150) configured for controlling the valve block and the heater such that said one function of said first and second function comprises receiving a fresh ammonia precursor solution whilst outputting a generated ammonia solution, at repetitive moments in time; and such that said other function comprises receiving the ammonia precursor solution before it enters said one compartment and/or receiving the generated ammonia solution leaving said one compartment.

2. The module of claim 1, wherein the other function of said first and second function is holding the ammonia precursor solution before it enters the compartment performing said one function and/or holding the generated ammonia solution leaving said compartment performing said one function.

3. The module of any one of the previous claims, wherein the heater extends in the first compartment.

4. The module of any one of the previous claims, wherein the first compartment has an outer circumferential wall, and wherein the second compartment surrounds the outer circumferential wall of the first compartment.

5. The module of any one of the previous claims, wherein the generated ammonia solution comprises ammonia, carbon dioxide and water;
wherein said one function is the second function, and
wherein the first function is a separating function, preferably separating the generated ammonia solution into a first ammonia rich fraction and a carbon dioxide rich fraction; said first ammonia rich fraction containing a smaller weight percentage of carbon dioxide than said solution and said carbon dioxide rich fraction containing a smaller weight percentage of ammonia than said solution.

6. The module of any one of the claims 1-4, wherein said one function is the first function, and said second function is a buffer function.

7. The module of claim 5, further comprising an output buffer compartment (20) surrounding at least partially the second compartment, said output buffer compartment being in fluid communication with the first compartment and being for holding the ammonia rich fraction leaving said first compartment.

8. The module of claim 5 or 7, further comprising an input buffer compartment (40) surrounding at least partially the second compartment, said input buffer compartment being in fluid communication with the second compartment and being for holding the ammonia precursor solution before entering the second compartment.

9. The module of claim 5, wherein the control module is for controlling the valve block and the heater such that the ammonia solution in said first compartment (30) is heated at a first temperature (T3); and the ammonia precursor solution in said second compartment (10) is heated at a second temperature (T1) lower than said first temperature.

10. The module of claim 7, wherein the valve block is for connecting the output buffer compartment (20) with the first compartment (30).

11. The module of claim 8, wherein the valve block is for connecting the input buffer compartment (40) with the second compartment (10).

12. The module of claim 10, wherein the control module is for controlling said valve block such that after a separating and decomposing step, an ammonia rich fraction is transferred from the first compartment (30) to the output buffer compartment (20), an ammonia solution is transferred from the second compartment (10) to the first compartment (30), and an ammonia precursor solution is transferred to the second compartment (10).

13. The module of claim 11 and 12, wherein the control module is further for controlling said valve block such that after a separating and decomposing step, an ammonia precursor solution is transferred to the input buffer compartment (40), and an ammonia precursor solution is transferred from said input buffer compartment (40) to said second compartment (10).

14. The module of any one of the previous claims, further comprising a pump arranged for pumping fluid between the first compartment and the second compartment.

15. The module of any one of the previous claims, wherein each compartment has a cylindrical inner and outer wall, and/or wherein the first and second compartments are concentric around the heater.

16. An ammonia precursor storage tank (200) comprising a module according to any one of the previous claims, wherein the module is integrated in the tank or in or on a wall of the tank, wherein said module is for allowing ammonia precursor solution stored in the tank to be transferred to one compartment of said first and second compartment, optionally via the other compartment of said first and second compartment.

17. An SCR system for a vehicle comprising a module according to any one of the claims 1-15 or a tank according to claim 16.

18. Fuel cell system comprising a module according to any one of the claims 1-15 or a tank according to claim 16.

## Patentansprüche

1. Modul (100) zur Verwendung an Bord eines Fahrzeugs, wobei das Modul ein Heizelement (50) und mindestens eine erste und eine zweite Kammer (10; 20; 30; 40; 20', 40') aufweist; wobei die erste Kammer das Heizelement (50) zumindest teilweise umgibt und die zweite Kammer die erste Kammer zumindest teilweise umgibt; wobei die erste Kammer zum Ausführen einer ersten Funktion in einem ersten Temperaturbereich dient und die zweite Kammer zum Ausführen einer zweiten Funktion in einem zweiten Temperaturbereich dient, wobei der zweite Temperaturbereich niedriger als der erste Temperaturbereich ist; wobei die erste Kammer in Fluidverbindung mit der zweiten Kammer steht; wobei eine Funktion der ersten oder zweiten Funktion das Zersetzen eines Ammoniakvorläufers unter Verwendung eines Katalysators zur Erzeugung einer Ammoniaklösung ist; wobei das Modul ferner einen Ventilblock (70) aufweist, der zum Verbinden mindestens der ersten und der zweiten Kammer konfiguriert ist, und ein Steuermodul (150), das zum Steuern des Ventilblocks und des Heizelements derart konfiguriert ist, dass die eine Funktion der ersten und der zweiten Funktion das Empfangen einer frischen Ammoniakvorläuferlösung aufweist, während eine erzeugte Ammoniaklösung zu sich wiederholenden Zeitpunkten ausgegeben wird; und derart, dass die andere Funktion das Empfangen der Ammoniakvorläuferlösung vor ihrem Eintritt in die eine Kammer und / oder das Empfangen der erzeugten Ammoniaklösung, die die eine Kammer verlässt, aufweist.

2. Modul nach Anspruch 1, wobei die andere Funktion der ersten und zweiten Funktion darin besteht, die Ammoniakvorläuferlösung zu halten, bevor sie in die Kammer eintritt, die die eine Funktion ausführt, und / oder die erzeugte Ammoniaklösung zu halten, die die Kammer verlässt, die die eine Funktion ausführt.

3. Modul nach einem der vorhergehenden Ansprüche, wobei sich das Heizelement in die erste Kammer erstreckt.

4. Modul nach einem der vorhergehenden Ansprüche, wobei die erste Kammer eine Außenumfangswand aufweist, und wobei die zweite Kammer die Außenumfangswand der ersten Kammer umgibt.

5. Modul nach einem der vorhergehenden Ansprüche, wobei die erzeugte Ammoniaklösung Ammoniak, Kohlendioxid und Wasser aufweist; wobei die eine Funktion die zweite Funktion ist, und wobei die erste Funktion eine Trennfunktion ist, wobei vorzugsweise die erzeugte Ammoniaklösung in eine erste ammoniakreiche Fraktion und eine kohlendioxidreiche Fraktion getrennt wird; wobei die erste ammoniakreiche Fraktion einen geringeren Gewichtsprozentsatz an Kohlendioxid als die Lösung enthält und die kohlendioxidreiche Fraktion einen geringeren Gewichtsprozentsatz an Ammoniak als die Lösung enthält.

6. Modul nach einem der Ansprüche 1 bis 4, wobei die eine Funktion die erste Funktion ist und die zweite Funktion eine Pufferfunktion ist.

7. Modul nach Anspruch 5, ferner aufweisend eine Ausgangspufferkammer (20), die das zweite Fach zumindest teilweise umgibt, wobei die Ausgangspufferkammer mit der ersten Kammer in Fluidverbindung steht und zum Halten der die erste Kammer verlassenden ammoniakreichen Fraktion dient.

8. Modul nach Anspruch 5 oder 7, ferner aufweisend eine Eingangspufferkammer (40), die die zweite Kammer zumindest teilweise umgibt, wobei die Eingangspufferkammer in Fluidverbindung mit der zweiten Kammer steht und zum Halten der Ammoniakvorläuferlösung vor dem Eintritt in die zweite Kammer dient.

9. Modul nach Anspruch 5, wobei das Steuermodul den Ventilblock und das Heizelement derart steuert, dass die Ammoniaklösung in der ersten Kammer (30) auf eine erste Temperatur (T3) erwärmt wird; und die Ammoniakvorläuferlösung in der zweiten Kammer (10) auf eine zweite Temperatur (T1) erwärmt wird, die niedriger als die erste Temperatur ist.

10. Modul nach Anspruch 7, wobei der Ventilblock zum Verbinden der Ausgangspufferkammer (20) mit der ersten Kammer (30) dient.

11. Modul nach Anspruch 8, wobei der Ventilblock zum Verbinden der Eingangspufferkammer (40) mit der zweiten Kammer (10) dient.

12. Modul nach Anspruch 10, wobei das Steuermodul den Ventilblock so steuert, dass nach einem Trenn- und Zersetzungsschritt eine ammoniakreiche Fraktion von der ersten Kammer (30) in die Ausgangspufferkammer (20) überführt wird, eine Ammoniaklösung von der zweiten Kammer (10) in die erste Kammer (30) überführt wird, und eine Ammoniakvorläuferlösung in die zweite Kammer (10) überführt wird.

13. Modul nach Anspruch 11 und 12, wobei das Steuermodul ferner zum Steuern des Ventilblocks derart vorgesehen ist, dass nach einem Trenn- und Zersetzungsschritt eine Ammoniakvorläuferlösung in die Eingangspufferkammer (40) übertragen wird und eine Ammoniakvorläuferlösung von der Eingangspufferkammer (40) zu der zweiten Kammer (10) übertragen wird.

14. Modul nach einem der vorhergehenden Ansprüche, ferner aufweisend eine Pumpe, die zum Pumpen von Fluid zwischen der ersten Kammer und der zweiten Kammer angeordnet ist.

15. Modul nach einem der vorhergehenden Ansprüche, wobei jede Kammer eine zylindrische Innen- und Außenwand aufweist und / oder wobei die erste und die zweite Kammer konzentrisch um das Heizelement herum angeordnet sind.

16. Ammoniakvorläufer-Speichertank (200), der ein Modul nach einem der vorhergehenden Ansprüche aufweist, wobei das Modul in den Tank oder in oder an eine Wand des Tanks integriert ist, wobei das Modul dazu dient, dass die Ammoniakvorläufer-Lösung, die in dem Tank gespeichert ist, in eine Kammer der ersten und zweiten Kammer wahlweise über die andere Kammer der ersten und der zweiten Kammer überführt wird.

17. SCR-System für ein Fahrzeug, aufweisend ein Modul nach einem der Ansprüche 1 bis 5 oder einen Tank nach Anspruch 16.

18. Brennstoffzellensystem, aufweisend ein Modul nach einem der Ansprüche 1 bis 15 oder einen Tank nach Anspruch 16.

## Revendications

1. Un module (100) destiné à être utilisé à bord d'un véhicule, ledit module comprenant un appareil de chauffage (50), et au moins un premier et un deuxième compartiment de rangement (10 ; 20 ; 30 ; 40 ; 20', 40') ;
le premier compartiment entourant au moins partiellement ledit appareil de chauffage (50), et le deuxième compartiment entourant au moins partiellement le premier compartiment ;
le premier compartiment étant destiné à mettre en oeuvre une première fonction dans une première gamme de températures, et le deuxième compartiment étant destiné à mettre en oeuvre une deuxième fonction dans une deuxième gamme de températures, ladite deuxième gamme de températures étant inférieure à ladite première gamme de températures ;
le premier compartiment étant en communication fluidique avec le deuxième compartiment ;
une fonction parmi ladite première ou deuxième fonction étant la décomposition d'un précurseur d'ammoniac en utilisant un catalyseur pour générer une solution d'ammoniac ;
le module comprenant en outre un bloc (70) formant vanne configuré pour connecter au moins lesdits premier et deuxième compartiments, et un module de commande (150) configuré pour commander le bloc formant vanne et l'appareil de chauffage de telle sorte que ladite une fonction parmi lesdites première et deuxième fonctions comprend le fait de recevoir une solution fraîche de précurseur d'ammoniac tout en délivrant une solution d'ammoniac générée, à intervalles réguliers ; et de telle sorte que ladite autre fonction comprend le fait de recevoir la solution de précurseur d'ammoniac avant qu'elle n'entre dans ledit un compartiment et/ou à recevoir la solution d'ammoniac générée sortant dudit un compartiment.

2. Le module selon la revendication 1, dans lequel ladite autre fonction parmi lesdites première et deuxième fonctions est le fait de contenir la solution de précurseur d'ammoniac avant qu'elle n'entre dans le compartiment effectuant ladite première fonction et/ou de contenir la solution d'ammoniac générée quittant ledit compartiment mettant en oeuvre ladite première fonction.

3. Le module selon l'une quelconque des revendications précédentes, dans lequel l'appareil de chauffage s'étend dans le premier compartiment.

4. Le module selon l'une quelconque des revendications précédentes, dans lequel le premier compartiment a une paroi circonférentielle extérieure, et dans lequel le deuxième compartiment entoure la paroi circonférentielle extérieure du premier compartiment.

5. Le module selon l'une quelconque des revendications précédentes, dans lequel la solution d'ammoniac générée comprend de l'ammoniac, du dioxyde de carbone et de l'eau ;
ladite une fonction étant la deuxième fonction, et
la première fonction étant une fonction de séparation, de préférence la séparation de la solution d'ammoniac générée en une première fraction riche en ammoniac et une fraction riche en dioxyde de carbone ; ladite première fraction riche en ammoniac contenant un pourcentage en poids de dioxyde de carbone inférieur à celui de ladite solution et ladite fraction riche en dioxyde de carbone contenant un pourcentage en poids d'ammoniac inférieur à celui de ladite solution.

6. Le module selon l'une quelconque des revendications 1 à 4, dans lequel ladite une fonction est la première fonction, et ladite deuxième fonction est une fonction de tampon.

7. Le module selon la revendication 5, comprenant en outre un compartiment (20) formant tampon de sortie entourant au moins partiellement le deuxième compartiment, ledit compartiment formant tampon de sortie étant en communication fluidique avec le premier compartiment et étant destiné à contenir la fraction riche en ammoniac sortant dudit premier compartiment.

8. Le module selon la revendication 5 ou selon la revendication 7, comprenant en outre un compartiment (40) formant tampon d'entrée entourant au moins partiellement le deuxième compartiment, ledit compartiment formant tampon d'entrée étant en communication fluidique avec le deuxième compartiment et étant destiné à contenir la solution de précurseur d'ammoniac avant d'entrer dans le deuxième compartiment.

9. Le module selon la revendication 5, dans lequel le module de commande est destiné à commander le bloc formant vanne et l'appareil de chauffage de telle sorte que la solution d'ammoniac dans ledit premier compartiment (30) est chauffée à une première température (T3) ; et la solution de précurseur d'ammoniac dans ledit deuxième compartiment (10) est chauffée à une deuxième température (T1) inférieure à ladite première température.

10. Le module selon la revendication 7, dans lequel le bloc formant vanne étant destiné à relier le compartiment (20) formant tampon de sortie au premier compartiment (30).

11. Le module selon la revendication 8, dans lequel le bloc formant vanne est destiné à relier le compartiment (40) formant tampon d'entrée au deuxième compartiment (10).

12. Le module selon la revendication 10, dans lequel le module de commande est destiné à commander ledit bloc formant vanne de telle sorte qu'après une étape de séparation et de décomposition, une fraction riche en ammoniac est transférée du premier compartiment (30) au compartiment (20) formant tampon de sortie, une solution d'ammoniac est transférée du deuxième compartiment (10) au premier compartiment (30) et une solution de précurseur d'ammoniac est transférée dans le deuxième compartiment (10).

13. Le module selon la revendication 11 et la revendication 12, dans lequel le module de commande est en outre destiné à commander ledit bloc formant vanne de telle sorte qu'après une étape de séparation et de décomposition, une solution de précurseur d'ammoniac est transférée dans le compartiment (40) formant tampon d'entrée et une solution de précurseur d'ammoniac est transférée dudit compartiment (40) formant tampon d'entrée dans ledit deuxième compartiment (10).

14. Le module selon l'une quelconque des revendications précédentes, comprenant en outre une pompe agencée pour pomper du fluide entre le premier compartiment et le deuxième compartiment.

15. Le module selon l'une quelconque des revendications précédentes, dans lequel chaque compartiment a une paroi intérieure et extérieure cylindrique, et/ou dans lequel les premier et deuxième compartiments sont concentriques autour de l'appareil de chauffage.

16. Un réservoir (200) de stockage de précurseur d'ammoniac comprenant un module selon l'une quelconque des revendications précédentes, le module étant intégré dans le réservoir ou dans ou sur une paroi du réservoir, ledit module étant destiné à permettre le transfert de la solution de précurseur d'ammoniac stockée dans le réservoir vers un compartiment parmi lesdits premier et deuxième compartiments, optionnellement via l'autre compartiment parmi lesdits premier et deuxième compartiments.

17. Un système SCR pour un véhicule comprenant un module selon l'une quelconque des revendications 1 à 15 ou un réservoir selon la revendication 16.

18. Système de pile à combustible comprenant un module selon l'une quelconque des revendications 1 à 15 ou un réservoir selon la revendication 16.
